# EUROPEAN PATENT APPLICATION

(11) **EP 4 623 892 A1**
(43) Date of publication of application: **01.10.2025**
(21) Application number: 24165836.8
(22) Date of filing: 25.03.2024
(51) Int. Cl.: A61K 8/34, A61K 8/49, A61K 8/60, A61Q 5/08, A61Q 5/10

(54) **ACTIVE AGENT FOR USE IN THE TREATMENT OF HAIR PIGMENTATION**

(71) Applicant: CUTANEON - Skin & Hair Innovations GmbH, 22335 Hamburg (DE)
(72) Inventor: CHÉRET, Jérémy, 13125 Berlin (DE); PAUS, Ralf, 22339 Hamburg (DE); AGRAMUNT MORENO, Juliá, 43870 Tarragona (ES)
(74) Representative: WSL Patentanwälte Partnerschaft mbB

(57) **Abstract**

In order to provide a new agent for hair pigmentation treatment in subjects, the present invention suggests an active agent for use in the treatment of hair pigmentation, wherein the active agent activates, enhances, inactivates, blocks or dampens the cellular response of the taste receptor TAS2R50 or interferes with the expression of said receptor. Furthermore, the present invention is directed to the use of said active agent as a medicament, a cosmetic or a food supplement in therapeutical or non-therapeutical treatment of hair pigmentation. In addition, the invention is directed to a corresponding cosmetic or medical hair pigmentation regulating or modulating composition or food supplement.

## Description

The present invention is directed to an active agent for use in the treatment of hair pigmentation, wherein said active agent activates, enhances, inactivates, blocks or dampens the cellular response of the taste receptor TAS2R50 or interferes with the expression of said receptor. Further, the present invention is directed to the use of said active agent as a medicament, a cosmetic or as a food supplement. In addition, the invention is directed to a corresponding cosmetic or medical hair pigmentation regulating or modulating composition or food supplement.

Hair greying is a highly prevalent condition present in about 50% of the general population. The prevalence of hair greying is 14.8% in those aged 30-34 years and 97.2% in those aged 60 years and above. In older individuals, grey hair might imply a subtle impact, where many individuals opt to cover them with tints. On young people, grey hair can have significant psychological impacts, sometimes close to the effects of baldness. This factor makes anti-grey hair products increasingly popular and influential in the hair care product market.

Grey hair is primarily caused by a reduction in melanin, the pigment being responsible for hair color. While aging is the most common reason for greying hair, several other factors can contribute to hair greying, namely genetic premature greying, extreme stress or traumatic events (telogen effluvium) or nutritional deficiencies in vitamins and minerals, particularly vitamin B12, iron, copper, and zinc. Further, there are a number of medical conditions being associated with hair greying, such as thyroid disorders (hyperthyroidism or hypothyroidism), vitiligo, autoimmune diseases, and alopecia areata.

So far, there is no definitive cure for grey hair available. However, some active agents are commonly used to potentially restore hair color, such as melanin supplements containing melanin precursors, vitamins (like biotin), minerals (like copper and zinc), and amino acids (like L-tyrosine) that may support melanin production. Some products contain antioxidants like vitamin E, vitamin C, and glutathione that shall neutralize free radicals and reduce oxidative stress.

In the continuous search for active agents for actively controlling and regulating hair pigmentation in subjects the inventors of the present invention found that the taste receptor TAS2R50 is involved in regulation and modulation of hair pigmentation.

Particularly, the inventors of the present application have found that activating or enhancing the cellular response of the taste receptor TAS2R50 enhances hair matrix melanogenesis in anagen phase hair follicles, characterized by increased melanocyte dendritic complexity, GP100 protein and alpha-melanocyte stimulating hormone (α-MSH) expression, and melanin production. The inventors concluded that the administration of an active agent that activates or enhances the cellular response of the taste receptor TAS2R50 or stimulates or promotes the expression said receptor is effective in repigmentation of grey hair.

On the other hand the inventors noted that an antagonist of the taste receptor TAS2R50 may be used for effecting a decrease in hair pigmentation or even for inducing total hair depigmentation, e.g. for use as an intentional hair whitening treatment.

In light of the above the present application discloses the use of an active agent for the treatment of hair pigmentation, wherein the active agent activates, enhances, inactivates, blocks or dampens the cellular re-sponse of the taste receptor TAS2R50 or interferes with the expression of said receptor.

A "taste receptor" describes a type of receptor that facilitates the sensation of taste in the oral cavity. Taste receptors are divided into two families, type 1 (sweet, TAS1R2 - TAS1R3), and type 2 (bitter, TAS2R1 - TAS2R50, and TAS2R60). Combinations of these receptors in dimers or other complexes contribute to different perceptions of taste.

The mRNA expression of some but not all TAS2Rs, including TAS2R3, TAS2R4, TAS2R5, TAS2R8, TAS2R9 TAS2R14, TAS2R30, TAS2R42, and TAS2R60 was detected in the RNA collected from full-thickness skin biopsies. Expression of bitter taste receptors TAS2R1 and TAS2R38 has been reported in human epidermal keratinocytes. A number of bitter taste receptor have been reported to be expressed in extraoral/nasal tissues, such as but not limited to the reproductive organs, the upper respiratory tract, the gastrointestinal tract, the brain and the immune system. EP 3 766 545 discloses the presence of TAS2R4 in hair follicle cells and its role in hair growth regulation.

"TAS2R50" is the official gene symbol for "Taste receptor type 2 subfamily R member 50" and identifies the protein that is encoded by the TAS2R50 gene in humans (NCBI Gene ID: 259296; HGNC: 18882; NCBI mRNA sequence: NM_176890.2; NCBI protein sequence: NP_795371.2).

TAS2R50 is a bitter taste receptor being ectopically expressed in different human tissues such as the lungs and skin. TAS2R50 mRNA has been identified in human scalp skin at the transcript level.

According to a specific embodiment the inventive active agent to be used in the treatment of hair pigmentation is an "agonist" of the taste receptor TAS2R50, that is a substance that binds to the receptor and activates or enhances the receptor to produce the cellular response, thereby effecting an increase in hair pigmentation. In specific embodiments of the invention the active agent is selected from any one of the TAS2R50 activating agonists andrographolide, amarogentin, isosinensetin and resveratrol.

According to some other embodiments the inventive active agent to be used in the treatment of hair pigmentation is an "antagonist" of the taste receptor TAS2R50, thereby effecting a decrease in hair pigmentation. In specific embodiments of the invention the active agent is siRNA interfering with the expression of TAS2R50.

Even though the inventors are not aware of any such instance, it cannot be excluded, that there exist prior art hair pigmentation treatment compositions comprising one of the specific inventive active agents mentioned above as an ingredient, without attributing said ingredient any effect on TAS2R50. In this event, such accidentally anticipated active agent may be excluded from the scope of the invention by a corresponding disclaimer. Except for excluding said accidentally anticipated specific active agent said disclaimer shall not further affect the scope of the claims.

Non-limiting examples for active agents being accidentally anticipated in the prior art are
- chemical compounds being attributed no medical, pharmacological or biological activity at all by the corresponding prior art,
- chemical compounds being attributed another medical, pharmacological or biological activity by the corresponding prior art as compared to the herein disclosed specific inventive active agents, and/or
- chemical compounds being attributed the same medical, pharmacological or biological activity by the corresponding prior art as compared to the herein disclosed specific inventive active agents, but not being directed to TAS2R50 but being directed to another target (e.g. receptor, enzyme, hormone, metabolite).

In some embodiments of the invention the TAS2R50 agonist is an aptamer binding to the TAS2R50 receptor and activating or enhancing the receptor to produce the cellular response, wherein the term "aptamer" as used herein refers to DNA, RNA or XNA oligonucleotide or peptide molecules that bind to a specific target molecule, such as receptor molecules.

According to an alternative of the present invention, an active agent that interferes with the expression of the taste receptor TAS2R50 is used in hair pigmentation treatment. An active agent that interferes with the expression of the taste receptor TAS2R50 is either miRNA, siRNA or a ribozyme targeted to the TAS2R50 gene or targeted to the mRNA corresponding to the TAS2R50 gene.

The term "miRNA" refers to microRNA, i.e. small non-coding RNA molecules containing 18 to 25 nucleotides and functioning in RNA knock-down and post-transcriptional regulation of gene expression. miRNAs function via base-pairing with complementary sequences within mRNA molecules. As a result, these mRNA molecules are knocked-down, by cleavage, destabilization and/or less efficient translation of the mRNA

The term "siRNA" refers to small interfering RNA, i.e. small non-coding RNA molecules, 18 to 25 base pairs in length. siRNA interferes with the expression of specific genes with complementary nucleotide sequences by degrading mRNA after transcription, thereby preventing translation.

According to the present invention a gene is "targeted" by a miRNA, siRNA or a ribozyme when the miRNA, siRNA or a ribozyme molecule selectively decreases or inhibits the expression of TAS2R50. The phrase "selectively decrease or inhibit" as used herein refers to miRNA, siRNA or a ribozyme that affects the expression of TAS2R50.

In specific embodiments of the invention miRNA or siRNA interfere with the gene expression of the taste receptor TAS2R50 by hybridizing under stringent conditions to the gene transcript, i.e. the TAS2R50 mRNA, wherein hybridizing "under stringent conditions" means annealing to the target mRNA region, under standard conditions, e.g., high temperature (e.g. < 60 °C for 2 hours) and/or low salt content (e.g. 0.1xSSC) which tend to disfavor hybridization.

In some embodiments of the invention the active agent is an agent acting on a co-receptor to TAS2R50 resulting in stimulation or promotion of expression of the receptor TAS2R50.

According to the present invention the above-mentioned active agents are used in the treatment of hair pigmentation, either in order to stop or slow down the depigmentation process or even to achieve repigmentation of partially or totally depigmented hair or in order to effect a decrease in hair pigmentation or even to induce total hair depigmentation, e.g. for intentional hair whitening treatment.

In specific embodiments of the invention the treatment is effected locally, i.e. in, on or at the skin area, where the affected hair follicles are located. In some embodiments of the invention said treatment is effected topically, wherein the term "topical" refers to a medication that is applied to a particular place on the skin and/or its appendages, such as hair. In specific embodiments the topical application is epicutaneous, meaning that the agonist or antagonist is applied directly to the skin. In other embodiments of the invention the treatment is effected transdermally, wherein the term "transdermal" refers to a medication that is applied across the Stratum corneum into the deeper skin layers, e.g. by injection with a standard needle or microneedles. In other embodiments of the invention the treatment is effected transappend-ageally, wherein the term "transappendageal" refers to an applied formulation that is permeating the skin via skin appendage structures (such as the hair follicles, sebaceous glands, and sweat glands) into the deeper skin layers. In yet other embodiments of the invention the treatment is effected by oral administration in the form of e.g. capsules or tablets or in the form of food supplement drinks.

The term "treatment" as used herein refers to any action resulting in the change of a physical condition. Particularly, the "treatment of hair depigmentation" refers to any loss of an initial hair pigmentation condition, such as hair greying. In contrast to that the term "treatment of hair pigmentation" refers to effecting a decrease in hair pigmentation or even to inducing total hair depigmentation.

In specific embodiments of the invention the above-mentioned active agent is used as a cosmetic in the treatment of hair pigmentation. Particularly, the use as a cosmetic occurs non-therapeutically, but in order to achieve a change of an initial hair pigmentation condition, such as age-related or premature hair greying. In some embodiments the cosmetic is used for intentional hair whitening treatment.

In those embodiments, where the above-mentioned active agent is used as a cosmetic the active agent used should be cosmetically acceptable, wherein "cosmetically acceptable" means that the active agent should not be toxic or harmful or have any other detrimental side effects upon application on hair and/or skin.

In some specific embodiments of the invention the above-mentioned active agent is used as a food supplement for hair repigmentation.

In other specific embodiments of the invention the above-mentioned active agent is used as a medicament in the topical treatment of hair pigmentation disorder, wherein the term "disorder" refers to any functional abnormality or disturbance of the normal healthy condition, and the term "medicament" refers to a substance useful in curing, treating or preventing a condition of disorder.

In some embodiments the above-mentioned active agent is used as a medicamentin the treatment of a hair pigmentation disorder being selected from the group consisting of poliosis circumscripta, post-inflammatory depigmentation, post-infection depigmentation, post-blister depigmentation, post-burn depigmentation, post-traumatic depigmentation, post-birth depigmented stretch marks [striae distensae], post-bleaching depigmentation.

In those embodiments, where the above-mentioned active agent is used as a medicament the active agent used should be pharmaceutically acceptable, wherein "pharmaceutically acceptable" means that the active agent should not be toxic or harmful or have any other detrimental side effects upon application on hair and/or skin.

In some embodiments at least one of the inventive active agents is used as an ingredient of a composition for use as a cosmetic or food supplement or medicament in the treatment of hair pigmentation said composition further comprising at least one auxiliary agent selected from the group consisting of carriers, recipients, adjuvants, diluents, penetration enhancers and disintegrants.

In specific embodiments of such compositions the auxiliary agent is selected from the group consisting of liposomes, nanoparticles, carboxymethyl cellulose, hydroxyethyl cellulose, mineral oil, petrolatum, glycerin, polysorbat 80, hydroxyethyl starch, dextran, and polyethylene glycol.

In the inventive compositions the concentration of the active agent usually is in the range of from 10 to 10.000 µM. In some embodiments the lower limit for the concentration of the active agent is 30 µM or even 100 µM. In some embodiments the upper limit is 3.000 µM or 1.000 µm. This results in preferred ranges of e.g. from 30 to 10.000 µM, from 30 to 3.000 µM, from 10 to 3.000 µM, 100 to 3.000 µM and the like.

Referring to the total weight of an inventive composition the concentration of the active agent may vary in specific embodiments within the range of from 0.001 to 30 wt.-%. In some embodiments the lower limit is 0.01 wt.-%, 0,1 wt.-% or even 1 wt.-%. In some embodiments the upper limit is 20 wt.-%, 10 wt.-% or 5 wt.-%. This results in preferred ranges of e.g. from 0.001 to 10 wt.-%, from 0.01 to 5 wt.-%, from 0.01 to 20 wt.-%, from 1 to 30 wt.-% and the like.

Specific embodiments of the inventive compositions comprise one of the inventive active agents or combinations of at least two of said inventive active agents. Irrespective, whether the composition comprises just one or two or more of the inventive active agents, in some embodiments, the composition further comprise at least one other common active agent being effective in the treatment of hair repigmentation.

In such embodiments the other active agent may for example be selected from melanin, melanin precursors, biotin, folic acid, p-aminobenzoic acid, copper, zinc, L-tyrosine, vitamin E, vitamin C, and glutathione.

Generally, the inventive composition may be used in any formulation suitable for treatment of hair repigmentation. In specific embodiments of the invention the composition is formulated in the form of an ointment, a lotion, a cream, a shampoo, a gel, a solution, an emulsion, a suspension, a spray, a capsulse, a tablet, a plaster or a sustained release plaster. Liquid application forms may be applied by means of a microneedle device or prior to sonication, electrical stimulation, etc.. In some embodiments liquid application forms may be food supplement drinks. Further alternative routes of administration are intraoculary and intranasally.

As mentioned above, the inventive use of the above-mentioned active agent may also occur non-therapeutically, wherein non-therapeutically refers to a treatment not being directed to curing, treating or preventing a condition of disorder (see above).

Therefore, the present invention is further directed to a non-therapeutic method of hair repigmentation, wherein an effective amount of at least one of the above-mentioned active agent is administered to a subject.

The non-therapeutic method also encloses embodiments, where the above-mentioned active agent is administered to the subject to be treated simultaneously, sequentially or separately together with at least one other active agent useful in the treatment of hair repigmentation (see above).

The following examples show some of the features of specific embodiments of the present invention. However, the skilled reader will understand that those embodiments are just exemplary but do not restrict the inventive idea to exactly the features or the combination of features of the embodiments of the examples.

In the description of the examples it is referred to the following figures, wherein
- Figure 1: shows experimental data according to which treatment with the inventive active agent andrographolide and amarogeting increases melanocyte dendritic complexity in pigmented HFs,
- Figure 2: shows experimental data according to which treatment with the inventive active agent andrographolide and amarogentin increases expression of the melanocyte marker GP100 in pigmented, non-grey HFs,
- Figure 3: shows experimental data according to which treatment with the inventive active agent andrographolide and amarogentin increases melanin production in pigmented, non-grey HFs,
- Figure 4: shows experimental data according to which treatment with the inventive active agent andrographolide and amarogentin upregulates TAS2R50 in pigmented , non-grey HFs,
- Figure 5: shows microscopic photographs according to which treatment with the inventive active agent andrographolide results in melanocytes producing fine lines of melanin in grey/white HFs,
- Figure 6: shows experimental data according to which treatment with the inventive active agent andrographolide enhances melanin synthesis and α-MSH expression in melanocytes of grey/white HFs,

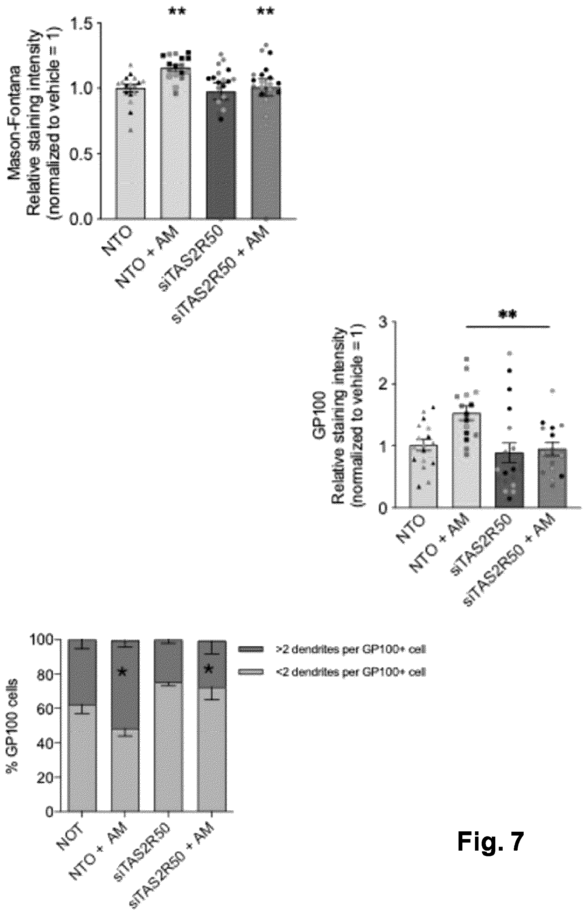

- Figure 7: shows experimental data according to which treatment with the inventive active agent amarogentin with the presence of siRNA against TAS2R50 (siTAS2R52 + AM) prevents the melanin synthesis and the increase melanocyte dendritic complexity compared to inactivated siRNA (NTO + AM) in pigmented, non-grey HFs, and

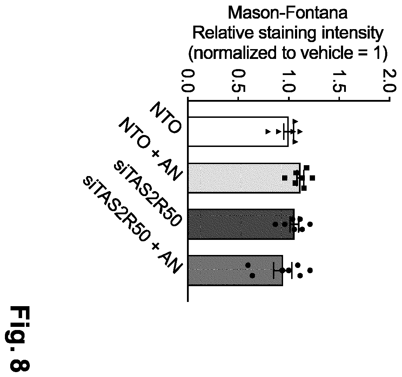

- Figure 8: shows experimental data according to which treatment with the inventive active agent andrographolide with the presence of siRNA against TAS2R50 (siTAS2R52 + AN) prevents the melanin synthesis compared to inactivated siRNA (NTO + AM) in pigmented, non-grey HFs.

### Examples

### 1. Human Follicle Organ Culture experiments

Initially, we utilized an antibody against TAS2R50 in freshly isolated human scalp hair follicles (HFs) to confirm the receptor presence in the basal ORS compartment of the HF. To validate our antibody, we used human lung airway smooth muscle (ASM) tissue as a positive control, observing cells around ASM expressing the receptor, consistent with previous findings at the transcript level (see above). The results showed TAS2R50 protein expression in both, human ASM tissue and human HFs (data not shown).

Next, we investigated if the TAS2R50 agonist, namely andrographolide (AN) and amaorengint (AM) have an effect on hair follicle melanogenesis. Thus, we employed GP100 and Mason Fontana melanin quantification on the 6-day Human Follicle Organ Culture (HFOC) condition with 50µM AN or 200 µM AM.

Due to differences in melanin amounts between anagen and catagen HFs (pigmentation is active only during anagen), just pigemented anagen HFs were considered for these analyses. Thus, we excluded any grey/white and catagen HFs.

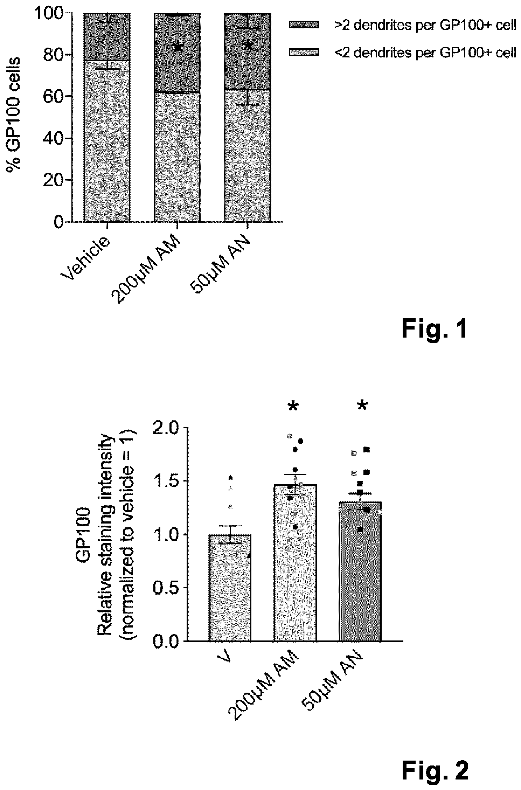

We found that anagen HFs showed highly developed dendritic melanocytes with melanocytes having more than 2 dendrites per cell body, more GP100 protein expression, and more melanin production compared to vehicle (Figures 1-3).

Percentage indicates melanocytes with less than or more than 2 dendrites. Intensity quantification was performed by using GP100 and MF histochemistry (GP: Mean ± SEM, n = 24-28 per group from 2-3 donors; MF: Mean ± SEM, n = 11-14 per group from 2-3 donors).

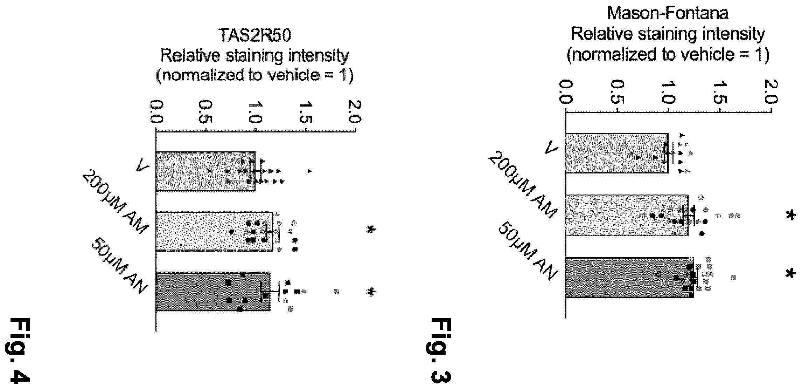

Further, we quantified the protein expression of TAS2R50 after AN or AM incubation to find that there is a significant increase of TAS2R50 protein expression in the ORS suggesting AN and AM treatment upregulates TAS2R50 (Figure 4).

In summary, AN and AM enhanced hair follicle melanogenesis in anagen HFs, characterized by increased melanocyte dendritic complexity, GP100 protein expression, and melanin production. These findings suggest that AN and AM enhances hair follicle melanogenesis in human HFs.

### 2. Hair repigmentation experiment with white/grey HFs employing AN

We incubated 20 grey/white hairs from 2 patients with widespread hair greying on the scalp with 50µM AN or vehicle during a 6-day period. Afterward, we performed qIHM in the pigmentary unit where amelanotic and melanotic melanocytes reside and conducted a melanin production and α-MSH expression analysis using WS staining.

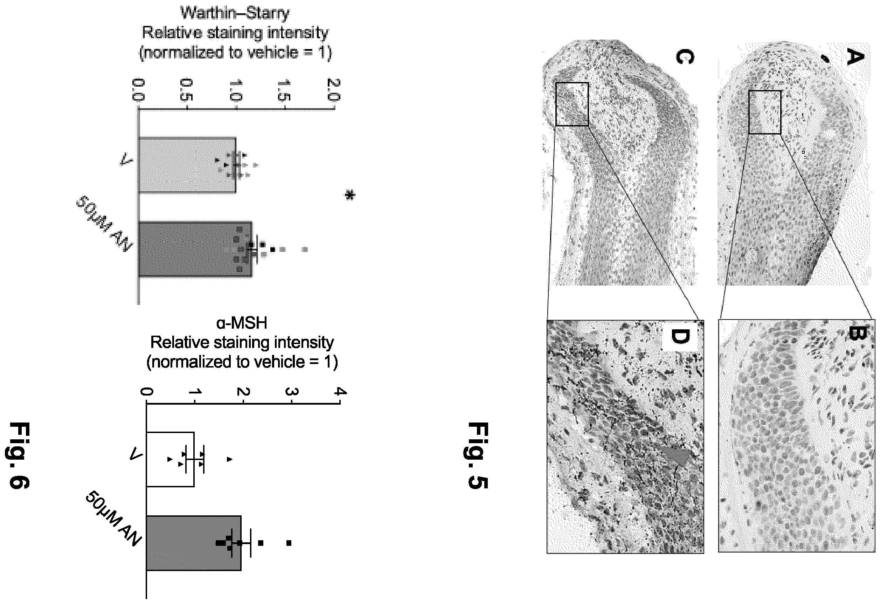

After this 6-day organ culture, we found a significant increase in melanin production and α-MSH expression in anagen hairs incubated with AN compared to the ones in the control group (Figures 5 and 6). It is possible to observe that melanocytes have started to produce melanin granules, as depicted by the arrow in Figure 5D, which are absent in the anagen hair from the control group (Figure 5B). Thus, AN is capable of facilitating melanin synthesis in HF melanocytes, which shows that this active agent helps to reverse/delay hair greying.

### 3. Effect of TAS2R50-siRNA

To assess the TAS2R50-dependence of the previously mentioned effects in the specified embodiments, we re-stimulated the HFs with AM and AN in the presence of TAS2R50 siRNA (siTAS2R50) within HFOC, applying our established gene silencing protocols.

We found that neither AM nor AN elicited the previously observed effects when co-incubated with siTAS2R50 (NTO + AM/AN vs siTAS2R50 + AM/AN). Moreover, we observed a further reduction in pigmentation and a decrease in melanocyte dendritic complexity when siTAS2R50 was incubated without any drug (NTO vs siTAS2R50). We conclude, that the silencing of the receptors alone, without AN or AM, can reduce pigmentation.

Taken together, these findings demonstrate that the phenomena induced by AM and AN were specifically mediated by TAS2R50 signaling.

## Claims

1. Active agent for therapeutical use in the treatment of hair depigmentation, wherein said active agent activates or enhances the cellular response of the taste receptor TAS2R50 or stimulates or promotes the expression of said receptor.

2. Active agent for therapeutical use in the treatment of hair depigmentation according to claim 1, wherein the active agent is an agonist of the taste receptor TAS2R50.

3. Active agent for therapeutical use in the treatment of hair depigmentation according to claim 1 or 2, wherein said active agent is selected from
a) any one of the TAS2R50 activating agonists andrographolide, amarogentin, isosinensetin and resveratrol, or an aptamer binding to TAS2R50 and activating or enhancing said receptor to produce a cellular response,
b) miRNA, siRNA or a ribozyme targeted to the TAS2R50 gene or targeted to the mRNA corresponding to the TAS2R50 gene, or
c) an agent acting on a co-receptor to TAS2R50 resulting in stimulation or promotion of expression of the receptor TAS2R50.

4. Active agent for therapeutical use in the treatment of hair depigmentation according to one of the claims 1 to 3, wherein the treatment is for treating a depigmentation disorder being selected from poliosis circumscripta, post-inflammatory depigmentation, post-infection depigmentation, post-blister depigmentation, post-burn depigmentation, post-traumatic depigmentation, post-birth depigmented stretch marks [striae distensae], post-bleaching depigmentation.

5. Use of an active agent as a cosmetic or food supplement for the non-therapeutical treatment of hair pigmentation, wherein the active agent activates, enhances, inactivates, blocks or dampens the cellular response of the taste receptor TAS2R50 or interferes with the expression of said receptor.

6. Use of an active agent as a cosmetic or food supplement for the non-therapeutical treatment of hair pigmentation according to claim 5, wherein the active agent is an agonist of the taste receptor TAS2R50, thereby effecting an increase in hair pigmentation.

7. Use of an active agent as a cosmetic or food supplement for the non-therapeutical treatment of hair pigmentation according to claim 5, wherein the active agent is an antagonist of the taste receptor TAS2R50, thereby effecting a decrease in hair pigmentation.

8. Use of an active agent as a cosmetic or food supplement for the non-therapeutical treatment of hair pigmentation according to claim 5, wherein said active agent is selected from
a) any one of the TAS2R50 activating agonists andrographolide, amarogentin, isosinensetin and resveratrol, or an aptamer binding to TAS2R50 and activating or enhancing said receptor to produce a cellular response,
b) miRNA, siRNA or a ribozyme targeted to the TAS2R50 gene or targeted to the mRNA corresponding to the TAS2R50 gene, or
c) an agent acting on a co-receptor to TAS2R50 resulting in stimulation or promotion of expression of the receptor TAS2R50.

9. Cosmetic or medical hair pigmentation regulating composition or food supplement comprising at least one active agent according to one of the claims 1 to 8 and at least one auxiliary agent selected from the group consisting of carriers, excipients, adjuvants, diluents, penetration enhancers, and disintegrants.

10. Cosmetic or medical hair pigmentation regulating composition or food supplement according to claim 9, wherein the auxiliary agent is selected from the group consisting of liposomes, nanoparticles, carboxymethyl cellulose, hydroxyethyl cellulose, mineral oil, petrolatum, glycerin, polysorbate 80, hydroxyethyl starch, dextran, and polyethylene glycol.

11. Cosmetic or medical hair pigmentation regulating composition or food supplement according to claim 9 or claim 10, further comprising at least one other active agent being effective in the treatment of hair depigmentation.

12. Cosmetic or medical hair pigmentation regulating composition or food supplement according to one of the claims 9 to 11, wherein the composition is formulated in the form of an ointment, a lotion, a cream, a shampoo, a gel, a solution, an emulsion, a suspension, a spray, a capsulse, a tablet, a plaster or a sustained release plaster.

13. Non-therapeutic method of hair pigmentation regulation, wherein an effective amount of at least one active agent that activates, enhances, inactivates, blocks or dampens the cellular response of the taste receptor TAS2R50 or interferes with the expression of said receptor or an agent acting on a co-receptor to TAS2R50 modulating the expression of the receptor TAS2R50 is administered to a subject.

14. Non-therapeutic method of hair pigmentation regulation according to claim 13, wherein said active agent is selected from
a) any one of the TAS2R50 activating agonists andrographolide, amarogentin, isosinensetin and resveratrol, or an aptamer binding to TAS2R50 and activating or enhancing said receptor to produce a cellular response,
b) miRNA, siRNA or a ribozyme targeted to the TAS2R50 gene or targeted to the mRNA corresponding to the TAS2R50 gene, or
c) an agent acting on a co-receptor to TAS2R50 resulting in stimulation or-promotion of expression of the receptor TAS2R50.
